# EUROPÄISCHE PATENTSCHRIFT

(11) **EP 1 915 207 B1**
(45) Veröffentlichungstag und Bekanntmachung des Hinweises auf die Patenterteilung: **19.10.2011**
(21) Anmeldenummer: 06776207.0
(22) Anmeldetag: 12.07.2006
(51) Int. Cl.: B01J 23/648, B01J 27/053, B01J 27/195, B01J 37/02, C07C 5/27, C07C 2/08, C07C 2/54, B01J 35/10

(54) **MEHRSCHICHTKATALYSATOR AUF NIOBBASIS ZUR KATALYTISCHEN UMSETZUNG VON KOHLENWASSERSTOFFEN**
MULTI-LAYER CATALYST MADE FROM NIOBIUM FOR THE CATALYTIC CONVERSION OF HYDROCARBONS
CATALYSEUR MULTICOUCHE A BASE DE NIOBIUM UTILISE POUR LA CONVERSION CATALYTIQUE D'HYDROCARBURES

(30) Priorität: 13.07.2005 DE 102005032723
(43) Veröffentlichungstag der Anmeldung: 30.04.2008
(73) Patentinhaber: SÜD-CHEMIE AG, 80333 München (DE)
(72) Erfinder: SCHMIDT, Friedrich, 83024 Rosenheim (DE)
(74) Vertreter: Stolmár, Matthias
(86) Internationale Anmeldenummer: PCT/EP2006/006844
(87) Internationale Veröffentlichungsnummer: WO 2007/006571

(56) Entgegenhaltungen:
- WO-A-2006/048180
- US-A1- 2003 092 566
- ARANDA D.A.G. ET AL: "Characterization and dehydrogenation activity of Pt/Nb2O5 catalysts" CATALYSIS TODAY, AMSTERDAM, NL, Bd. 28, 1996, Seiten 119-125, XP002406919
- TOKIO IIZUKA ET AL: "ACIDIC AND CATALYTIC PROPERTIES OF NIOBIUM PENTAOXIDE" BULLETIN OF THE CHEMICAL SOCIETY OF JAPAN, CHEMICAL SOCIETY OF JAPAN, TOKYO, JP, Bd. 56, Nr. 10, 1. Oktober 1983 (1983-10-01), Seiten 2927-2931, XP002406920 ISSN: 0009-2673

## Beschreibung

Die vorliegende Erfindung betrifft einen festen, sauren Katalysator umfassend mehrere chemisch unterschiedliche Schichten auf der Basis eines Kerns umfassend Oxide der V. Nebengruppe, der für die säurekatalysierte Umlagerung von Kohlenwasserstoffen wie beispielsweise für die Hydroisomerisierung von Paraffinen geeignet ist. Weiter betrifft die vorliegende Erfindung ein Verfahren zur Herstellung eines derartigen Katalysators sowie die Verwendung des Katalysators.

Zu den wichtigsten Raffinerieprozessen, die durch feste Säuren katalysiert werden, zählen beispielsweise das katalytische Spalten schwerer Kohlenwasserstofffraktionen, das hydrierende Spalten von schweren Kohlenwasserstoffen, das Entfernen von Wachsen aus Kerosin und Dieselfraktionen, die Alkylierung von C4-Fraktionen und die Hydroisomerisierung von C4- bis C7-Fraktionen sowie im Bereich der Petrochemie beispielsweise die Alkylierung oder die Acylierung von Aromaten oder die Dealkylierung, Transalkylierung und Isomerisierung von Alkylaromaten. Weitere wichtige chemische Prozesse, die durch feste Säuren katalysiert werden, sind die Veresterung, die Veretherung, die Aminierung oder die Dehydratisierung von Alkoholen, die Dehydratisierung von Olefinen und die Oligomerisierung von funtionalisierten Kohlenwasserstoffen. Dabei gewinnen vor allem die beiden Raffinierprozesse der Alkylierung von C4-Fraktionen sowie die Isomerisierung höherer Paraffine als wichtige Alternative zur Erhöhung der Oktanzahl im Fahrbenzin vermehrt an Bedeutung.

Seit einiger Zeit wird versucht, die beiden vorerwähnten Prozesse anstatt mit korrosiven festen Säuren, wie beispielsweise chloriertem Aluminiumoxid, mit festen aber nicht korrosiven Säuren wie z.B. Zeolithen oder sulfatiertem Zirkonoxid durchzuführen.

Üblicherweise werden für die nicht korrosive Hydroisomerisierung von Paraffinen als Zeolithkatalysatoren Mordenite eingesetzt. Diese liefern jedoch erst bei Temperaturen um 250°C die gewünschten Ausbeuten, jedoch ist die Selektivität der erzielten Isomere nicht ideal. Erheblich höhere Selektivitäten der gewünschten Isomere werden dagegen nur bei deutlich tieferer Temperatur erreicht. Bei dieser Temperatur ist der Umsatz der Mordenitkatalysatoren verglichen mit Katalysatoren aus chloriertem Aluminiumoxid zu gering. Der Nachteil von chloriertem Aluminiumoxid ist seine extreme Empfindlichkeit selbst gegenüber Spuren von Wasser, was einen erhöhten Aufwand zur Reinigung der Ausgangsstoffe bedingt.

Katalysatoren auf der Basis von sulfatiertem Zirkonoxid zeigen (siehe beispielsweise EP 1 002 579 A1) optimale Selektivitäten bei Temperaturen, die unter denen liegen, bei denen Zeolithbasierte Katalysatoren eingesetzt werden, die aber noch deutlich oberhalb der Temperaturen liegen, bei denen mit chloriertem Aluminiumoxid gearbeitet wird. Katalysatoren auf der Basis von Zirkonoxid bedürfen daher der zusätzlichen Promotion mit Elementen wie Eisen, Mangan oder Nickel. Selbst diese dotierten bzw. promotierten Katalysatoren erreichen nicht die gewünschten Selektivitäten von Katalysatoren auf der Basis von chloriertem Aluminiumoxid.

Niobverbindungen und Materialien stellen mittlerweile eine Alternative für einige Anwendungen zu den üblichen Titan- bzw. Zirkonkatalysatoren dar (Niobium, Science and Technology, Proceedings Int. Symposium Juli 2001, Orlando, Florida, Nibobium 2001 Limited, Richville, S. 269 ff.). Insbesondere in Bezug auf seine Elektronnegativität und Mechanismen der katalytischen Reaktionen gibt es große Unterschiede zwischen Niob und seinen im Periodensystem benachbart angeordneten Elementen wie Vanadium, Zirkon und Molybdän. Die katalytische Wirkung von Niobverbindungen ist ziemlich unterschiedlich von denjenigen dieser Elemente, und daher nicht vorhersehbar.

Es ist bekannt, dass Nioboxide die katalytische Aktivität verbessern und die Lebensdauer des Katalysators verlängern, wenn geringe Mengen an Nioboxiden zu bekannten Katalysatoren zugegeben werden. Hydriertes Niobpentoxid, Nb₂O₅ · nH₂O, das üblicherweise auch Niobsäure genannt wird, und auch Niobphosphate sind starke Säuren und werden deswegen auch als Zusatz für feste saure Katalysatoren verwendet.

Da Niobsäure schon Wasser enthält, ist die Wasserempfindlichkeit von Niobsäure verglichen beispielsweise mit chloriertem Aluminiumoxid äußerst niedrig.

Die Säurestärke von Niobsäure (Ho = -5,6) entspricht etwa 70 % der Säurestärke von H₂SO₄, wenn Niobsäure bei Temperaturen von ungefähr 100 bis 300°C kalziniert wird, obgleich die Oberfläche von Niobsäure, die bei 500°C kalziniert wurde, im Allgemeinen neutral ist.

Das US-Patent Nr. 4,652,544 offenbart einen festen Katalysator bestehend im Wesentlichen aus hydratisiertem Nioboxid, bei dem die Säurestärke der Katalysatoroberfläche durch Zusatz von Phosphoriger Säure auf seiner Oberfläche erhöht wurde. Damit wurde außerdem die Kristallisation des Nioboxids gehemmt und der Verlust in der katalytischen Aktivität nach Behandlung bei hoher Temperatur verzögert. Die Herstellung dieses Katalysators erfolgt, indem man hydratisiertes Nioboxid oder ein Anhydrid davon mit einer Phosphorigen Säure behandelt. Dieser Katalysator hydratisiert Ethylen zu Ethanol.

Weitere Anwendungen von Niobionen und Niobverbindungen als Katalyator oder Katalysatorkomponenten sind in den Übersichtsartikeln von J.C. Vedrine, Catalysis Today, 28, 1996, 3-15 "Niobium Oxide based Materials as Catalysts for acidic and partial Oxidation Type Reactions" sowie von F. A. Chernyshkova, Russian Chemical Review, Volume 62, 1993, Nr. 8, S. 743 - 749 "Niobic acid,- a new Heterogeneous Catalyst for Processes in Petrochemical and Organic Synthesis" zusammengefasst. Generell werden Niobverbindungen in der Katalyse entweder als zusätzliche Komponenten für schon bestehende Katalysatoren verwendet sowie für die partielle Oxidation von Kohlenwasserstoffen.

Des weiteren offenbart das US-Patent Nr. 5,668,074 einen Lewis-sauren Niobkatalyator der hochkorrosiv ist. Dieser Katalysator wird für die Isomerisierung von Alkanen und Cycloalkanen verwendet und wird durch das Aufbringen von Niob oder Tantalverbindungen auf ein bereits reduziertes Vorprodukt von Platin bzw. Palladium auf Tonerde hergestellt. Dieser Niob enthaltende Vorläufer wird in einem nicht reduzierenden Gas und mindestens eine Fluoralkan bzw. Chlorfluoralkan, vorzugsweise erhitzt.

Ein Nachteil aller bisher beschriebener nicht zeolithischer Katalysatoren ist deren Empfindlichkeit gegenüber Verunreinigungen von Wasser im Ausgangsstrom.

Der vorliegenden Erfindung liegt daher die Aufgabe zugrunde, einen Katalysator zur Verfügung zu stellen, der auf einer festen Säure basiert und insbesondere gegenüber Spuren von Wasser in der Reaktionsmischung, insbesondere bei der katalytischen Umsetzung von Kohlenwasserstoffen, wie beispielsweise der Hydroisomerisierung von Paraffinen unempfindlicher ist als die bislang bekannten Katalysatoren, beispielsweise auf der Basis von chloriertem Aluminiumoxid oder promotiertem Zirkonoxid. Weiterhin soll dieser Katalysator ebenso eine vorzügliche Tieftemperaturaktivität und eine hohe Selektivität wie die bislang bekannten Katalysatoren aufweisen.

Diese Aufgabe wird gelöst durch einen Mehrschichtkatalysator gemäß Anspruch 1.

Die Trägerkomponente enthält entweder eine Niob- oder Tantalverbindung. Bevorzugt umfaßt die Trägerkomponente eine Niobverbindung, ganz besonders bevorzugt Niobsäure, die weitgehend wasserunempfindlich ist und dennoch eine hohe Säurestärke aufweist. Der erfindungsgemäße Katalysator kann als besonders saurer Katalysator bezeichnet werden, der aus spezifisch definierten Schichten besteht und einen erhöhten Umsatz, Stabilität sowie eine Selektivität insbesondere bei der Isomerisierung von leichtem Naphtha zur Erhöhung seines Isoparaffingehalts ermöglicht.

In ganz besonders bevorzugten Ausführungsformen enthält die Trägerkomponente eine mit einem Erdalkalimetall oder mit einem Lanthanoid dotierte Niobsäure, was die Säurestärke der Niobsäure noch weiter erhöht.

In einer vorteilhaften Ausführungsform enthält die Trägerkomponente weiter ein Bindemittel, beispielsweise eine Aluminium- , Silizium- oder Zirkonverbindung, bzw. deren gemischten und nicht gemischten Oxiden, Sulfaten oder Hydroxiden, die die Säurestärke des Katalysatorkerns weiter erhöht. Beispiele für das erfindungsgemäße Bindemittel sind neben Siliziumdioxid beispielsweise Zirkoniumoxid, Aluminiumoxid, Böhmit oder ein Aluminat. Weiterhin ermöglicht das Bindemittel das Herstellen von Formkörpern aus den Trägerkomponenten, so dass auch verschiedene geometrische Formen für einen erfindungsgemäßen Mehrschichtkatalysator einfach zu realisieren sind.

In einer ganz besonders bevorzugten Ausführungsform des erfindungsgemäßen Katalysators besteht der Kern des erfindungsgemäßen Mehrschichtkatalysators aus Nioboxid, das weiterhin ein Bindemittel wie beispielsweise Tonerde oder Böhmit enthält. Der Anteil an Bindemittel beträgt bevorzugt 5 - 70 Gew%, bezogen auf das Gesamtgewicht eines erfindungsgemäßen Katalysators. Im Allgemeinen ist bevorzugt, dass das Bindemittel ebenso die Säurestärke erhöht.

Zwar kann der Katalysator auch in Pulverform verwendet werden, die Zugabe des erfindungsgemäßen Bindemittels führt insbesondere bei der Verarbeitung zu Formkörpern zu einer Erhöhung der mechanischen Stabilität und zu einer besseren Handhabung als beim Pressen zu Formkörpern ohne Bindemittel.

Es wurde weiter gefunden, dass die Aktivität und die Selektivität eines erfindungsgemäßen Katalysators gesteigert werden kann, wenn eine weitere Schicht auf der Trägerkomponente aus einer sogenannten Promotorverbindung vorhanden ist. Die Promotorkomponente wild ausgewählt aus (NH₄)₂SO₄, und NH₄Fe(SO₄)₂ sowie Mischungen davon. Ganz besonders bevorzugt ist die Promotorverbindung in einer Menge von 5 - 50 Gew% bezogen auf das Gesamtgewicht am Katalysator in dem erfindungsgemäßen Katalysator enthalten.

Auf dieser Schicht aus einer Promotorverbindung ist eine weitere Schicht umfassend eine Verbindung der Platinmetalle, insbesondere einer Verbindung aus Platin oder Palladium aufgebracht in einer Menge von 0,01 bis 3 Gew% bezogen auf das Gesamtgewicht des erfindungsgemäßen Katalysators, um die Aktivität und Selektivität des erfindungsgemäßen Katalysators noch weiter zu steigern. Neben Platin und Palladium sind weiter bevorzugt Platinmetalle wie Ruthenium, Rhodium, Palladium, Iridium und Platin. Dabei ist die Verwendung von Platin und seinen Verbindungen für Hydroisomerisierungen besonders bevorzugt.

Es wurde gefunden, dass der erfindungsgemäße Katalysator insbesondere für die Umwandlung von Kohlenwasserstoffen, wie beispielsweise Hydroisomerisierung von Paraffinen geeignet ist, da der erfindungsgemäße Katalysator ermöglicht, die Hydroisomerisierung bei geringen Temperaturen von 140°C - 180°C durchzuführen, wobei ein hoher Umsatz am Gleichgewicht und sehr gute Selektivitäten erzielt werden Es wird eine Steigerung der "Research Octane Number" gegenüber dem industriellen Standard eines Zeolith basierten Katalysators um bis zu 5 Punkte erzielt.

Durch diese weitere, bevorzugt an der äußeren Oberfläche des Katalysators angeordneten, Schicht, die eine Verbindung der Platinmetalle umfasst, ist auch der Einsatz des erfindungsgemäßen Katalysators für Hydrierungen, Dehydrierungen und Hydroisomerisierungen besonders vorteilhaft möglich.

Der vorliegend verwendete Begriff "Schicht" umfasst dabei sowohl diskrete, physikalisch unterscheidbare Schichten, wie auch ineinander übergehende Lagen bzw. Schichten, wie sie z.B. durch aufeinander folgendes Tränken eines Formkörpers mit Lösungen unterschiedlicher Verbindungen entstehen können.

Die Aufgabe der vorliegenden Erfindung wird weiter durch ein Verfahren zur Herstellung des Mehrschichtkatalysators gelöst, umfassend die Schritte des
a) Lösens einer Niob-Sauerstoffverbindung
b) anschließenden Zugebens einer verdünnten Säure bis zur Ausfällung von Niobsäure.
d) des Trocknens der Niobsäure
e) des Herstellens eines Formkörpers aus der getrockneten Niobsäure
f) des Aufbringens des Promotorkomponente auf den Formkörper
g) des Aufbringens einer Verbindung der Platinmetalle.

Bevorzugt wird weiterhin in Schritt a) eine Templatverbindung zugegeben, die beispielsweise eine quartäre Ammoniumverbindung, nicht ionisches Polyethylenoxid oder ein Flüssigkristall sein kann, so dass die Niobsäure mit einer gezielten Porosität ausgefällt werden kann, was einen zusätzlichen vorteilhaften synergistischen Effekt liefert. Durch eine höhere Mesoporosität wird eine bessere Zugänglichkeit der Edukte zu den aktiven Zentren und eine kürzere Verweilzeit des Zwischenproduktes erreicht und damit eine Weiterreaktion (Oligomerisierung) unterdrückt.

Bevorzugt wird die getrocknete Niobsäure weiterhin kalziniert. Außerdem wird nach jedem Aufbringen einer weiteren Schicht der Formkörper getrocknet und/oder erneut kalziniert. Eine Zwischenkalzinierung liefert oftmals eine höhere Stabilität der Katalysatorleistung.

Das erfindungsgemäße Verfahren wird beispielsweise bevorzugt als Ionenaustauschverfahren eines Alkaliniobats, beispielsweise Kaliumniobat K₄Nb₆O₁₇ mit 0,5 M HNO₃ oder Schwefelsäure in wässriger Lösung bei Raumtemperatur zur H₄Nb₆O₁₇ mit einem atomaren Verhältnis von H:Nb = 2:3 durchgeführt. Alternativ dazu kann ein Niobat, wie beispielsweise KCa₂Nb₃0₁ₒ, zu HCa₂Nb₃O₁₆ in einem Verhältnis von H:Nb = 1:3 umgesetzt werden. Selbstverständlich können ebenso andere Erdalkali- und/oder Lanthanidmodifizierte Verbindungen, deren Protonengehalt (Verhältnis von H:Nb) und Säurestärke (abhängig von der chemischen Zusammensetzung) dem zu katalysierenden Prozess angepasst sind, verwendet werden.

Ebenso kann aus kommerziell erhältlicher Niobsäure, die ggf. modifiziert wird durch Quellung mit Hilfe von wie vorstehend erläuterten organischen Templaten und anschließender Fällung mit verdünnten Säuren das erfindungsgemäße Verfahren durchgeführt werden.

Die Formgebung im Rahmen des erfindungsgemäßen Verfahrens kann auf verschiedene Art und Weise erfolgen, wie beispielsweise durch Pressen oder Extrudieren. Dabei wird das Pulver der NIobsäure nach Zusatz eines Bindemittels oder Bindemittelvorläufers, wie beispielsweise Pseudo-Böhmit und ggf. weitere Peptisierungsmittel und/oder andere Extrudierhilfsstoffe extrudiert. Die Extrudate können anschließend getrocknet werden, beispielsweise bei Temperaturen von 90°C bis 100°C. Gegebenenfalls können die Formkörper noch kalziniert werden, wobei im Allgemeinen Temperaturen im Bereich 350°C bis 600°C verwendet werden.

Im Anschluss an das optionale Trocknen und/oder Kalzinieren wird bevorzugt eine Schicht einer katalytisch aktiven Promotorkomponente aufgebracht, ausgewählt aus (NH₄)₂SO₄ und NH₄Fe(SO₄)₂ sowie Mischungen davon. Die Zugabe dieser Promotorkomponenten bzw. deren Aufbringen erfolgt auf beliebige, dem Fachmann an sich geläufige Weise, beispielsweise durch Vermischen, Aufbringen durch Rakeln, Bedampfen, Tränken oder Imprägnieren mit einer Lösung.

In bevorzugten Ausführungsformen wird der Formkörper beispielsweise mit einer entsprechenden Lösung der Promotorkomponente getränkt. Dabei kann die Lösung eine wässrige oder nichtwässrige sein. Die Tränklösung wird dabei vorzugsweise so eingestellt, dass die Tränklösung vollständig vom Katalysator aufgenommen wird. So entsteht im Innern des Katalysators ein Gradient der Konzentration der Promotorkomponente, so dass an der Oberfläche des so behandelten Formkörpers fast nur Promotorkomponente vorliegt. Die Katalysatoren werden anschließend beispielsweise bei Temperaturen von etwa 90°C bis 130°C für 5 bis 20 Stunden getrocknet und anschließend optional beispielsweise bei 350°C bis 400°C für 5 bis 10 Stunden kalziniert.

Im Anschluss an das Trocknen und/oder Kalzinieren, des Zwischenproduktes wird bevorzugt eine weitere Schicht eine katalytisch aktive Komponente aufgebracht, ausgewählt aus Verbindungen der Platingruppe. Auch hier erfolgt das Aufbringen der weiteren Schicht aus einer katalytisch aktiven Komponente durch jede dem Fachmann an sich geläufige Art, wie beispielsweise durch intensives Vermischen, Bedampfen, Rakeln, Tränken oder Imprägnieren mit einer Lösung.

Bevorzugt wird der Katalysator beispielsweise mit einer entsprechenden Lösung eines Platin metalls getränkt, beispielsweise mit einer wässrigen Lösung aus H₂PtCl₆ im Falle von Platin. Auch hier wird die Tränklösung vorzugsweise so eingestellt, dass die Tränklösung vom Katalysatorformkörper aufgenommen wird. Anschließend werden die Katalysatoren beispielsweise bei Temperaturen von 90 bis 130°C für 5 bis 20 Stunden getrocknet und anschließend bei 350°C bis 400°C für 5 bis 10 Stunden kalziniert.

Der so hergestellte erfindungsgemäße Katalysator eignet sich für alle säurekatalysierten Reaktionen, wie z.B. die Hydroisomerisierung von Paraffinen, das katalytische Spalten schwerer Kohlenwasserstofffraktionen, das Hydrierende Spalten von schweren Kohlenwasserstoffen, das Entfernen von Wachsen aus Kerosin und Dieselfraktionen, für die Alkylierung von C4-Fraktionen sowie im Bereich der Petrochemie beispielsweise für die Alkylierung oder Acylierung von Aromaten oder die Dealkylierung, Transalkylierung oder Isomerisierung von Alkylaromaten und im Bereich der übrigen chemischen Prozesse der Veresterung, Veretherung, Aminierung oder die Dehydratisierung von Alkoholen, die Hydratisierung von Olefinen, die Dimerisierung von Olefinen und die Oligomerisierung von funktionalisierten Kohlenwasserstoffen, das milde Cracken von Kohlenwasserstoffen, Entwachsen (dewaxing), die Hydroisomerisierung von Fischer-Tropsch-Fraktionen.

In einer besonders bevorzugten Ausführungsform eignet sich der derart hergestellte erfindungsgemäße Katalysator ganz besonders für die Hydroisomerisierung von C4- bis C7-Fraktionen und von Wachsen.

Der erfindungsgemäße Katalysator wird weiter bei der Umwandlung von Kohlenwasserstoffen verwendet. Dabei eignet sich der erfindungsgemäße Katalysator besonders gut zum Reformieren von Schnitten der Erdöldestillation, zum Erhöhen der Fließfähigkeit von Gasölen, zur Isomerisierung von Olefinen oder aromatischen Verbindungen, zum katalytischen oder hydrierenden Spalten wie auch zur Oligomerisierung oder Polymerisation von olefinischen oder acetylenischen Kohlenwasserstoffen. Weitere Anwendungen sind Alkylierungsreaktionen, Transalkylierung und Isomerisierung oder Disproportionierung von Aromaten und alkylsubstituierten Aromaten, Dehydrierungen oder Hydrierungen, Hydratisierung und Dehydratisierung, Alkylierung und Isomerisierung von Olefinen, Entschwefelung, Umsatz von Alkoholen und Ethern zu Kohlenwasserstoffen und Umsatz von Paraffinen oder Olefinen zu Aromaten.

Ganz besonders bevorzugt eignet sich der Katalysator zur Hydroisomerisierung von Naphthenen. Gegenstand der vorliegenden Erfindung ist daher insbesondere auch die Verwendung des erfindungsgemäßen Katalysators für die Hydroisomerisierung höherer Paraffine. Unter dem Begriff höhere Paraffine werden dabei gesättigte lineare Kohlenwasserstoffe mit einer Kohlenstoffanzahl von mehr als 4 Kohlenstoffatomen verstanden.

Die Hydroisomerisierung wird dabei in Gegenwart von Wasserstoff vorzugsweise bei Temperaturen unterhalb von 290°C, vorzugsweise bei etwa 80°C bis 260°C durchgeführt. Der Druck wird bei der Hydroisomerisierung vorzugsweise in einem Bereich von 1 bis 50 bar bei einer Flüssig-Raumgeschwindigkeit (LHSV) von etwa 0,1 bis 10 Liter pro Stunde des zugeführten Kohlenwasserstoffes bzw. des kohlenwasserstoffhaltigen Gemisches je Liter Katalysator durchgeführt.

Im nachfolgenden wird die Erfindung anhand von Ausführungsbeispielen näher erläutert.

### Beispiel 1: Erfindungsgemäße Herstellung eines Katalysatorvorläufers auf der Basis von Nioboxid.

113 g (0,323 mol Nb) Ammoniumnioboxalat NH₄[NbO(C₂O₄)₂(H₂O)₂]·(H₂O) (Molekulargewicht: 350,91 g/mol) werden bei Raumtemperatur in 1,0 Liter Wasser gelöst und die Lösung 2 Stunden bei Raumtemperatur gerührt. Dann werden 90 g Oxalsäure (Molekulargewicht: 90,035 g/mol) in 1 Liter Wasser bei Raumtemperatur gelöst. Unter Rühren wird darauf die Ammoniumnioboxalat Lösung in die Oxalsäurelösung überführt. Der entstehende Feststoff wird von der Lösung abfiltriert und anschließend solange mit 0,25 molarer Salpetersäure gewaschen, bis die Konzentration von Ammonium-Ionen im Filtrat kleiner als 10 ppm ist. Der Filterkuchen wird bei 120 °C unter Luftzutritt während 16 Stunden getrocknet.

### Beispiel 2: Erfindungsgemäße Herstellung von porösen Formkörpern eines Bindemittel-enthaltenden Katalysatorvorläufers auf der Basis von Nioboxid.

Für die Formgebung wird der getrocknete Filterkuchen mit einer Mühle auf eine mittlere Teilchengrüße von 500 µm gemahlen. Der getrocknete und gemahlene Filterkuchen wird dann mit 5 g eines handelsüblichen peptisierbaren Aluminiumoxidhydrates in einem Knetwerk 15 Minuten lang trocken gemischt. Zu dieser Mischung wird langsam 50 ml einer 1,5%-igen wässrigen Salpetersäurelösung und 5 ml Steatitöl gegeben und bis zur Bildung einer plastischen, formbaren Masse geknetet und dann in einem handelsüblichen Extruder zu Formkörpern mit einem Durchmesser von etwa 1,6 mm und einer Länge von etwa 5 mm extrudiert. Die Extrudate werden bei 120 °C 5 Stunden getrocknet und dann bei 350 °C 5 Stunden kalziniert.

### Beispiel 3 Promotierung

Der so hergestellte Katalysatorformkörper wird bei Raumtemperatur mit einer Lösung von 2 g Ammoniumsulfat (wasserfrei gerechnet) als Promotorkomponente in 50 ml Wasser unter ständigem Wenden besprüht. Danach wird der Katalysator bei 120 °C 15 Std getrocknet und dann bei 350°C 5 Stunden kalziniert.

### Beispiel 4 Herstellung des Platin-haltigen Katalysators

Zur erfindungsgemäßen Herstellung des Pt-haltigen Katalysators wurden 0.35 g einer Hexachloroplatin-sauren Lösung, die 30 wt.-% Pt in 10 ml Wasser enthält, zu den sulfathaltigen Extrudaten hinzugefügt. Die Mischung wurde mechanisch bei der Raumtemperatur gemischt und bei 120 C° 12 Stunden lang getrocknet. Der trockene Formkörper wird in Luft kalziniert mit 100 °C/h bis 350°C aufgeheizt und dort für 3 h gehalten und dann mit 100 °C/h abgekühlt.

### Beispiel 5

100 g eines nicht-ionischen Block-Copolymers P123 werden ((PEO)₂₀(PPO)₇₀(PEO)₂₀ in 1 Liter Propanol gelöst und dann langsam 1 Mol Niobiumchlorid (Molekulargewicht 270,17) hinzugefügt. Die Mischung wird bei 40°C unter Rühren über 10 Tage gealtert. Das Produkt wird abgetrennt und das Templat über 7 Stunden bei 450°C entfernt.

Der Katalysator wird wie in den Beispielen 2 bis 4 beschrieben weiterverarbeitet.

### Beispiel 6

100 g eines nicht-ionischen Block-Copolymers P123 werden ((PEO)₂₀(PPO)₂₀(PEO)₂₀ in 1 Liter Propanol gelöst und dann langsam 0,1 Mol Niobiumchlorid (Molekulargewicht 270,17) hinzugefügt. Zu der Mischung werden 0,1 mol Wasser hinzugefügt. Dann wird die Mischung bei 40°C unter Rühren über 10 Tage gealtert. Das Produkt wird abgetrennt und das Templat über 7 Stunden bei 450°C entfernt.

Der Katalysator wird wie in den Beispielen 2 bis 4 beschrieben weiterverarbeitet.

### Beispiel 7

100 g eines nicht-ionischen Block-Copolymers P85 werden ((PEO)₂₅(PPO)₄₀(PEO)₂₅ in 1 Liter Propanol gelöst und langsam 0,1 Mol Niobiumchlorid (Molekulargewicht 270,17) hinzugefügt. Zu der Mischung werden 0,1 mol Wasser hinzugefügt. Dann wird die Mischung bei 40°C unter Rühren über 10 Tage gealtert. Das Produkt wird abgetrennt und bei 80°C weitergealtert. Danach wird der Katalysatorvorläufer jeweils mit 1 Liter Wasser gewaschen, 5 Stunden bei 120°C getrocknet und dann 7 Stunden bei 450°C kalziniert.

Der Katalysator wird wie in den Beispielen 2 bis 4 beschrieben weiterverarbeitet.

### Beispiel 8

100 g eines nicht-ionischen Block-Copolymers P85 werden ((PEO)₂₅ (PPO)₄₀ (PEO)₂₅ in 1 Liter Propanol gelöst und dann langsam 0,1 Mol Niobiumchlorid (Molekulargewicht 270,17) hinzugefügt. Zu der Mischung werden 0,1 mol Wasser hinzugefügt. Dann wird die Mischung bei 40°C unter Rühren über 10 Tage gealtert.

Das Produkt wird abgetrennt und bei 80°C weitergealtert. Der Katalysatorvorläufer wird mit jeweils 1 Liter Wasser drei Mal gewaschen und dann 5 Stunden bei 120°C getrocknet. Anschließend wird der Katalysatorvorläufer 7 Stunden bei 350 °C kalziniert.

Der Katalysator wird wie in den Beispielen 2 bis 4 beschrieben weiterverarbeitet.

### Beispiel 9

1 Mol Ammoniumnioboxalat NH₄[NbO(C₂O₄)₂(H₂O)₂]·(H₂O) (Molekulargewicht: 350,91 g/mol) werden bei Raumtemperatur in 5 Liter Wasser gelöst und die Lösung 2 Stunden bei Raumtemperatur gerührt. Dann werden 3 Mol Oxalsäure (Molekulargewicht: 90,035 g/mol) in 5 Liter Wasser bei Raumtemperatur gelöst. Unter Rühren wird darauf die Ammoniumnioboxalat Lösung in die Oxalsäurelösung überführt. Der entstehende Feststoff wird von der Lösung abfiltriert und anschließend zu einer Lösung von 100 g eines nicht-ionischen Block-Copolymers P85 ((PEO)₂₅(PPO)₄₀(PEO)₂₅ gelöst in 1 Liter Propanol hinzugefügt. Dann wird die Mischung bei 40°C unter Rühren über 10 Tage gealtert. Das Produkt wird abgetrennt.und bei 80°C weitergealtert. Der Katalysatorvorläufer wird mit jeweils 1 Liter Wasser drei Mal gewaschen und dann 5 Stunden bei 120°C getrocknet. Anschließend wird der Katalysatorvorläufer 7 Stunden bei 350 °C kalziniert.

Der Katalysator wird wie in den Beispielen 2 bis 4 beschrieben weiterverarbeitet.

### Vergleichsbeispiel 1:

0.8 Kilogramm hydratisierte Niobsäure Nb₂O₅ ·nH2O (CBMM, Marke HY-340®) wird in einer Kugel-Mühle zu einer durchschnittlichen Teilchengröße von µm 500 gemahlen. Für das Formen des Katalysators werden 0.4 Kilogramm eines kommerziellen peptisierbaren Tonerdehydrats als Abbindemittel mit 0,8 Kilogramm der gemahlenen Niobsäure in einem Kneter für 15 Minuten trocken gemischt. Zu dieser Mischung wird langsam 0,1 Kilogramm einer 1.5 wt.-%-igen wässrigen Salpetersäurelösung und 10 ml Steatitöl bis zur Plastifizierung gegeben und geknetet. Danach wird die Masse durch eine Extrudierplatte gepresst, die Bohrungen enthält, so dass nach dem Trocknen die Extrudate einen Durchmesser von 1,5 mm haben. Die Extrudate werden bei °C 120 5 Stunden getrocknet und kalziniert dann bei °C 350 5 Stunden mit einer Rampe von 100°/h zu 350 °C, bei °C 350 (550°C, °C 450) für 4 h gehalten und dann abgekühlt mit 100 °C/h.

Eine Lösung von 0.35 g der Hexachloroplatin-sauren Lösung, die 30 wt.-% Pt in 10 ml Wasser enthält, wurde den kalzinierten Extrudaten hinzugefügt. Die Mischung wurde mechanisch bei Raumtemperatur gemischt und bei °C 120 12 Stunden lang getrocknet. Der trockene Formkörper wird in Luft kalziniert mit 100 °C/h bis 350°C (550°C, °C 450) dort für 3 h gehalten und mit 100 °C/h abgekühlt.

### Vergleichsbeispiel 2

800 g Zirkoniumhydroxid (MEL XZO632/03) werden in einem Kneter (Werner und Pfleiderer, Z-Kneter) mit 500 g Ammoniumzirkoniumcarbonat-Lösung (20 %, MEL) und 300 g Ammoniumsulfat gemischt und 10 min geknetet. Dann werden zu der flüssigen Mischung nacheinander unter Kneten 40 g Methocell (Zellulose-Ether, DOW), 50 g Steatitöl und 30 g VE-Wasser gegeben. Die erhaltene Mischung wird 45 min mit kalter Luft angeblasen, bis die Masse eine Konsistenz erreicht, damit sie extrudiert werden kann. Die erhaltene Mischung wird extrudiert (Extruder von Fujio Paudal Co. Ltd; Model EXKFs-1 Gechwindigkeitseinstellung 0,4). Der Durchmesser der Extrusions ist 1,6 mm. Die Extrudate werden 15 Stunden bei 80°C im Trockenschrank getrocknet und anschließend mit folgendem Temperaturprogramm kalziniert: (1) Aufheizen von Raumtemperatur auf 550°C mit einer Heizrate von 200°C/h, (2) Halten von 550°C für 3 Stunden, (3) Abkühlen von 550°C auf RT mit einer Rate von 200°C/h.

Die Extrudate werden mit einer Lösung von Hexachloroplatinsäure bei Raumtemperatur in der Weise getränkt, dass ein Pt-Gehalt auf dem fertig kalzinierten Katalysator von 0,5 Gew.-% erreicht wird.

Anschließend werden die Pt-getränkten Extrudaten 15 Stunden bei 80°C im Trockenschrank getrocknet und anschließend mit folgendem Temperaturprogramm kalziniert: (1) Aufheizen von Raumtemperatur auf 550°C mit einer Heizrate von 200°C/h, (2) Halten von 550°C für 3 Stunden, (3) Abkühlen von 550°C auf Raumtemperatur mit einer Rate von 200°C/h.

### Ausführungsbeispiel 1

Die gemäß den Beispielen 4 bis 9 sowie Vergleichsbeispielen 10 und 11 erhaltenen Pt-haltigen Katalysatoren wurden in einem Mikroreaktor mit reinem n-Pentan getestet. Die Testbedingungen waren wie folgt:

| | |
|---|---|
| Reaktordurchmesser | 8 mm |
| Katalysatorgewicht | 2,0 g |
| Katalysatorkorngröße (Siebfraktion des granulierten Materials | von 0,5 bis 1 mm |
| Druck | 20 bar |
| Temperatur | 120°C bis 250°C |
| Wasserstoff | 20,71 Nml/min |
| n-Pentan | 0,107 Nml/min |
| LHSV | 2 h-1 |
| H2:n-Pentan (molar) | 1:1 |

Die Analytik erfolgt mittels eines on-line GC, bei dem alle 15 Minuten ein Messpunkt erfasst wird.

Der Reaktor wurde wie folgt eingefahren: zuerst wurde Luft mit einer Geschwindigkeit von 33,33 ml/min eingeleitet, worauf der Reaktor von Raumtemperatur auf 350°C aufgeheizt wurde. Diese Temperatur wurde 1 Stunde gehalten und anschließend die Temperatur von 350°C auf 250°C abgesenkt. Dann wurde der Luftstrom unterbrochen und für 30 min durch einen Stickstoffstrom (33,33 ml/min) ersetzt. Der Stickstoffstrom wurde anschließend durch einen Wasserstoffstrom (33,33 ml/min) ersetzt. Dann wurde der Druck auf 30 bar H₂ erhöht und reines n-Pentan eingeleitet. Der Produktstrom wurde alle 30 Minuten gaschromatographisch analysiert. Nach 8 Stunden wurde die Temperatur auf 200°C gesenkt und der Katalysator 8 Stunden lang getestet, gefolgt von einer weiteren Abnahme der Temperatur auf 150°C und entsprechendem Test für 8 Stunden und schließlich wurde die Temperatur auf 250°C angehoben und der Katalysator 8 Stunden lang getestet, um eine ggf. erfolgende Katalysator Deaktivierung im Verlauf der Zeit aufzuklären.

### Definitionen:

i-C5-Aktivität:= Gew.-% i-C5^{p}/(Gew.-%i-C5^{p} + n-C5^{p})* 100;
%-Spaltprodukte:= Gew.-% C1-C4 Kohlenwasserstoffe
n.d.:= nicht nachweisbar

Die Ergebnisse für die Temperatur von 200°C sind im Folgenden aufgeführt:

| Katalysator | i-C5-Aktivität | %Spaltprodukte | Laufzeit Std.) |
|---|---|---|---|
| Beispiel 4 | 74,6 | n.d. | 120 |
| Beispiel 5 | 74,8 | 0,2 | 80 |
| Beispiel 6 | 75,0 | 0,3 | 80 |
| Beispiel 7 | 74,6 | 0,1 | 80 |
| Beispiel 8 | 75,5 | 0,05 | 80 |
| Beispiel 9 | 75,7 | n.d. | 120 |
| Beispiel 10 | 25 | 3,0 | 80 |
| Beispiel 11 | 72,4 | 2,5 | 80 |

Das Thermodynamische Gleichgewicht liegt bei einer i-C5-Aktivität von ca. 75,7%.

## Patentansprüche

1. Mehrschichtkatalysator zur katalytischen Umsetzung von Kohlenwasserstoffen umfassend
a) eine Trägerkomponente enthaltend ein dotiertes oder nicht dotiertes Oxid oder Hydroxid des Niobs oder Tantals oder Mischungen davon, darauf angeordnet
b) eine erste Schicht aus einer Promotorverbindung ausgewählt aus (NH₄)₂SO₄ und NH₄Fe(SO₄)₂ sowie Mischungen davon
c) eine weitere Schicht umfassend eine Verbindung der Platinmetalle.

2. Mehrschichtkatalysator nach Anspruch 1, wobei die Trägerkomponente weiter ein Bindemittel enthält.

3. Mehrschichtkatalysator nach Anspruch 2, wobei das Bindemittel eine Aluminium-, Silizium- oder Zirkoniumverbindung ausgewählt aus deren gemischten und nicht gemischten Oxiden, Sulfaten und Hydroxiden umfasst.

4. Mehrschichtkatalysator nach Anspruch 3, wobei die Menge an Bindemittel 5 - 70 Gew.-% bezogen auf das Gesamtgewicht an Katalysator beträgt.

5. Mehrschichtkatalysator nach einem der vorhergehenden Ansprüche, wobei die Menge an Promotorverbindung 5 - 50 Gew.-% bezogen auf das Gesamtgewicht an Katalysator beträgt.

6. Mehrschichtkatalysator nach einem der vorhergehenden Ansprüche, wobei die Menge an Platinmetallverbindung 0,01 bis 3 Gew.-% bezogen auf das Gesamtgewicht an Katalysator beträgt.

7. Mehrschichtkatalysator nach Anspruch 6, wobei die Platinmetallverbindung ausgewählt ist aus den Metallen, deren Legierungen untereinander sowie Oxiden der Platingruppenmetalle sowie Mischungen davon.

8. Mehrschichtkatalysator nach Anspruch 7, wobei die Platinmetallverbindung Platin ist.

9. Mehrschichtkatalysator nach einem der vorhergehenden Ansprüche, wobei die Trägerkomponente Niobsäure umfasst.

10. Mehrschichtkatalysator nach Anspruch 9, wobei die Trägerkomponente eine mit einem Erdalkalimetall oder Lanthanoid dotierte Niobsäure ist.

11. Verfahren zur Herstellung eines Mehrschichtkatalysators nach einem der vorhergehenden Ansprüche umfassend die Schritte des
a) Lösens einer Niob-Sauerstoffverbindung
b) anschließenden Zugebens einer verdünnten Säure bis zur Ausfällung von Niobsäure
c) Trocknens der Niobsäure
d) Herstellens eines Formkörpers aus der getrockneten Niobsäure
e) Aufbringens einer Promotorkomponente, ausgewählt aus (NH₄)₂SO₄ und NH₄Fe(SO₄)₂ sowie Mischungen davon, auf den Formkörper
f) Aufbringens einer Verbindung der Platinmetalle.

12. Verfahren nach Anspruch 11, wobei in Schritt a) weiter eine Templatverbindung zugegeben wird.

13. Verfahren nach Anspruch 12, wobei die Templatverbindung eine quartäre Ammoniumverbindung ist.

14. Verfahren nach einem der vorhergehenden Ansprüche, wobei die in Schritt c) getrocknete Niobsäure weiter kalziniert wird.

15. Verfahren nach einem der vorhergehenden Ansprüche, wobei vor Schritt d) ein Bindemittel zugegeben wird.

16. Verfahren nach Anspruch 15, wobei das Bindemittel ausgewählt ist aus Zirkonium-, Titan- und Aluminiümenthaltenden Verbindungen und Mischungen davon.

17. Verfahren nach einem der vorhergehenden Ansprüche, wobei der Mehrschichtkatalysator nach Schritt e) und/oder f) getrocknet und/oder kalziniert wird.

18. Verfahren nach einem der vorhergehenden Ansprüche, wobei die Platinmetallverbindung ausgewählt ist aus den Metallen, deren Legierungen untereinander sowie Oxiden der Platingruppenmetalle sowie Mischungen davon.

19. Verwendung des Katalysators nach einem der Ansprüche 1 bis 10 zur Umwandlung von Kohlenwasserstoffen.

20. Verwendung des Katalysators nach einem der Ansprüche 1 bis 10 zur Isomerisierung von Paraffinen.

21. Verwendung des Katalysators nach einem der Ansprüche 1 bis 10 zur Dimerisierung oder Oligomerisierung von Olefinen.

22. Verwendung des Katalysators nach einem der Ansprüche 1 bis 10 zur Spaltung von höheren Olefinen.

23. Verwendung des Katalysators nach einem der Ansprüche 1 bis 10 zur Reaktion von Olefinen mit Paraffinen zur Bildung von iso-Oktan.

## Claims

1. A multilayer catalyst for catalytic conversion of hydrocarbons, comprising
a)a support component comprising a doped or undoped oxide or hydroxide of an element of transition group V of the Periodic Table or mixtures thereof, arranged thereon
b)a first layer of a promoter compound selected from (NH₄)₂SO₄ and NH₄Fe(SO₄)₂ and mixtures thereof
c)a further layer comprising a compound of the platinum group metals.

2. The multilayer catalyst as claimed in claim 1, **characterized in that** the support component further comprises a binder.

3. The multilayer catalyst as claimed in claim 2, **characterized in that** the binder comprises an aluminum, silicon or zirconium compound selected from mixed and unmixed oxides, sulfates and hydroxides thereof.

4. The multilayer catalyst as claimed in claim 3, **characterized in that** the amount of binder is 5 - 70% by weight based on the total weight of catalyst.

5. The multilayer catalyst as claimed in one of the preceding claims, **characterized in that** the amount of promoter compound is 5 - 50% by weight based on the total weight of catalyst.

6. The multilayer catalyst as claimed in one of the preceding claims, **characterized in that** the amount of platinum metal compound is from 0.01 to 3% by weight based on the total weight of catalyst.

7. The multilayer catalyst as claimed in claim 6, **characterized in that** the platinum metal compound is selected from the metals, the alloys thereof with one another, and oxides of the platinum group metals and mixtures thereof.

8. The multilayer catalyst as claimed in claim 7, **characterized in that** the platinum metal compound is platinum.

9. The multilayer catalyst as claimed in one of the preceding claims, **characterized in that** the support component comprises niobic acid.

10. The multilayer catalyst as claimed in claim 9, **characterized in that** the support component is a niobic acid doped with an alkaline earth metal or lanthanoid.

11. A processs for preparing a multilayer catalyst as claimed in one of the preceding claims, comprising the steps of
a) dissolving a niobium-oxygen compound,
b) then adding a dilute acid until niobic acid precipitates,
c) drying the niobic acid,
d) preparing a shaped body from the dried niobic acid,
e) applying a promoter component, selected from (NH₄)₂SO₄ and NH₄Fe (SO₄)₂ to the shaped body,
f) applying a compound of the platinum group metals.

12. The process as claimed in claim 11, **characterized in that** a template compound is also added in step a).

13. The process as claimed in claim 12, **characterized in that** the template compound is a quaternary ammonium compound.

14. The process as claimed in one of the preceding claims, **characterized in that** the niobic acid dried in step c) is calcined further.

15. The process as claimed in one of the preceding claims, **characterized in that** a binder is added before step d).

16. The process as claimed in claim 15, **characterized in that** the binder is selected from zirconium-, titanium- and aluminum-containing compounds and mixtures thereof.

17. The process as claimed in one of the preceding claims, **characterized in that** the multilayer catalyst is dried and/or calcined after step e) and/or f).

18. The process as claimed in one of the preceding claims, **characterized in that** the platinum metal compound is selected from the metals, the alloys thereof with one another, and oxides of the platinum group metals and mixtures thereof.

19. The use of the catalyst as claimed in one of claims 1 to 10 for converting hydrocarbons.

20. The use of the catalyst as claimed in one of claims 1 to 10 for isomerizing paraffins.

21. The use of the catalyst as claimed in one of claims 1 to 10 for dimerizing or oligomerizing olefins.

22. The use of the catalyst as claimed in one of claims 1 to 10 for splitting higher olefins.

23. The use of the catalyst as claimed in one of claims 1 to 10 for reacting olefins with paraffins to form isooctane.

## Revendications

1. Catalyseur multicouche pour la conversion catalytique d'hydrocarbures comprenant
a) un composant support contenant un oxyde ou hydroxyde dopé ou non dopé de niobium ou de tantale ou de mélanges de ceux-ci, disposée sur celui-ci
b) une première couche d'un composé promoteur sélectionné parmi (NH₄)₂SO₄ et NH₄Fe(SO₄)₂ ainsi que des mélanges de ceux-ci
c) une autre couche comprenant un composé des métaux du groupe du platine.

2. Catalyseur multicouche selon la revendication 1, dans lequel le composant support contient en outre un liant.

3. Catalyseur multicouche selon la revendication 2, dans lequel le liant comprend un composé d'aluminium, de silicium ou de zirconium sélectionné parmi leurs oxydes, sulfates et hydroxydes mélangés et non mélangés.

4. Catalyseur multicouche selon la revendication 3, dans lequel la quantité de liant est de 5 à 70 % en poids par rapport au poids total du catalyseur.

5. Catalyseur multicouche selon l'une quelconque des revendications précédentes, dans lequel la quantité de composé promoteur est de 5 à 50 % en poids par rapport au poids total du catalyseur.

6. Catalyseur multicouche selon l'une quelconque des revendications précédentes, dans lequel la quantité de composé métallique du groupe du platine est de 0,01 à 3 % en poids par rapport au poids total du catalyseur.

7. Catalyseur multicouche selon la revendication 6, dans lequel le composé métallique du groupe du platine est sélectionné parmi les métaux, leurs alliages entre eux ainsi que des oxydes des métaux du groupe du platine et des mélanges de ceux-ci.

8. Catalyseur multicouche selon la revendication 7, dans lequel le composé métallique du groupe du platine est le platine.

9. Catalyseur multicouche selon l'une quelconque des revendications précédentes, dans lequel le composant support comprend de l'acide niobique.

10. Catalyseur multicouche selon la revendication 9, dans lequel le composant support est un acide niobique dopé avec un métal alcalinoterreux ou un lanthanoïde.

11. Procédé de fabrication d'un catalyseur multicouche selon l'une quelconque des revendications précédentes comprenant les étapes de
a) dissolution d'un composé de niobium-oxygène
b) addition subséquente d'un acide dilué jusqu'à précipitation d'acide niobique
c) séchage de l'acide niobique
d) fabrication d'un corps moulé à partir de l'acide niobique séché
e) application d'un composant promoteur sélectionné parmi (NH₄)₂SO₄ et NH₄Fe(SO₄)₂ ainsi que des mélanges de ceux-ci sur le corps moulé
f) application d'un composé des métaux du groupe du platine.

12. Procédé selon la revendication 11, dans lequel un composé gabarit est en outre ajouté à l'étape a).

13. Procédé selon la revendication 12, dans lequel le composé gabarit est un composé d'ammonium quaternaire.

14. Procédé selon l'une quelconque des revendications précédentes, dans lequel l'acide niobique séché à l'étape c) est en outre calciné.

15. Procédé selon l'une quelconque des revendications précédentes, dans lequel un liant est ajouté avant l'étape d).

16. Procédé selon la revendication 15, dans lequel le liant est sélectionné parmi des composés contenant du zirconium, du titane et de l'aluminium et des mélanges de ceux-ci.

17. Procédé selon l'une quelconque des revendications précédentes, dans lequel le catalyseur multicouche est séché et/ou calciné après l'étape e) et/ou f).

18. Procédé selon l'une quelconque des revendications précédentes, dans lequel le composé métallique du groupe du platine est sélectionné parmi les métaux, leurs alliages entre eux ainsi que des oxydes des métaux du groupe du platine et des mélanges de ceux-ci.

19. Utilisation du catalyseur selon l'une quelconque des revendications 1 à 10 pour la conversion d'hydrocarbures.

20. Utilisation du catalyseur selon l'une quelconque des revendications 1 à 10 pour l'isomérisation de paraffines.

21. Utilisation du catalyseur selon l'une quelconque des revendications 1 à 10 pour la dimérisation ou l'oligomérisation d'oléfines.

22. Utilisation du catalyseur selon l'une quelconque des revendications 1 à 10 pour le clivage d'oléfines supérieures.

23. Utilisation du catalyseur selon l'une quelconque des revendications 1 à 10 pour la réaction d'oléfines avec des paraffines en vue de la formation d'iso-octane.
